# EUROPÄISCHE PATENTANMELDUNG

(11) **EP 2 157 501 A1**
(43) Veröffentlichungstag der Anmeldung: **24.02.2010**
(21) Anmeldenummer: 08014786.1
(22) Anmeldetag: 20.08.2008
(51) Int. Cl.: G06F 3/048, A61B 3/06

(54) **Verfahren und Gerät zur Anpassung der für die Anzeige eines Objektes auf einer Anzeigevorrichtung verwendeten Farben**

(71) Anmelder: Cycos Aktiengesellschaft, 52477 Alsdorf (DE)
(72) Erfinder: Niederste-Hollenberg, Torsten, 52066 Aachen (DE)
(74) Vertreter: Fritzsche, Thomas

(57) **Zusammenfassung**

Die Erfindung betrifft ein Verfahren zur Anpassung der für die Anzeige eines Objektes auf einer Anzeigevorrichtung verwendeten Farben, wobei für einen jeweiligen Benutzer ein für diesen geeignetes Farbschema aus einer Mehrzahl von Farbschemata verwendet wird, und wobei mittels der Farbschemata die für die Anzeige von Objekten zu verwendenden Farbkontraste jeweils festgelegt werden. Dabei wird mittels der Anzeigevorrichtung eine erste Abfragemeldung mit zumindest zwei alternativen Eingabemöglichkeiten ausgegeben, wobei der Benutzer mittels der Abfragemeldung zu einer Eingabe aufgefordert wird, wobei eine erste der alternativen Eingabemöglichkeiten mittels eines ersten Farbkontrastes und eine zweite alternative Eingabemöglichkeit mittels eines davon abweichenden Farbkontrastes angezeigt wird, und wobei die Abfragemeldung derart gestaltet ist, dass der Benutzer zur Auswahl derjenigen der alternativen Eingabemöglichkeiten angeleitet wird, die für ihn erkennbar ist und/oder bevorzugt wird. Anhand der Eingabe des Benutzers wird ein geeignetes der Farbschemata eingestellt.

## Beschreibung

Die Erfindung betrifft ein Verfahren zur Anpassung der für die Anzeige eines Objektes auf eine Anzeigevorrichtung verwendeten Farben gemäß dem Patentanspruch 1, und ein Gerät zur Ausführung des Verfahrens gemäß dem Patentanspruch 6.

Computer und andere Mikroprozessor gesteuerte Geräte werden meist mittels textbasierter oder graphischer Benutzeroberflächen gesteuert, wobei anstelle monochromer Anzeigevorrichtungen heutzutage meist Farbdisplays zum Einsatz kommen. Während bei monochromen Anzeigen die Wahrnehmung eines Benutzers auf einem Hell-Dunkel-Kontrast beruht, wird bei den farblich gestalteten Benutzeroberflächen oft in erster Linie ein Farbkontrast wahrgenommen. Das hat den Vorteil, dass beispielsweise wichtige Bildschirminhalte besser herausgestellt werden können und durch den sinnvollen Einsatz verschiedener Farben die Informationsdichte erhöht werden kann.

Die bei der Darstellung verwendeten Farbkontraste, meist eine Kombination aus einer ersten Farbe für den Vordergrund und einer zweiten Farbe für den Hintergrund, werden von unterschiedlichen Benutzern oft unterschiedlich wahrgenommen, wobei beispielsweise einzelne Farbkontraste (Farbkombinationen) besser wahrgenommen werden können, als andere, oder aber einzelne Farbkontraste als angenehmer wahrgenommen werden, als andere.

Dieses Empfinden ist stark von der Wahrnehmung des jeweiligen Benutzers abhängig, so dass die von einem Hersteller von Software oft als "Standard" verwendeten Farbkontraste nicht gewünscht sind oder unbrauchbar sind.

Zur Lösung dieses Problems können bei vielen Computern und Geräten für jeden Benutzer unterschiedliche sogenannte Farbschemata eingestellt werden, in denen für ein Gerät, ein Programm oder für ein Betriebssystem aus einer Anzahl vordefinierter oder selbst-definierbarer Farbschemata ein gewünschtes bzw. geeignetes ausgewählt werden kann.

Es gibt Menschen, die unter einer Farbsehschwäche leiden, d.h., dass diese Personen einzelne oder mehrere Farbkontraste nicht oder nur unzureichend differenzieren können. Ein Beispiel ist die sogenannte "Rot-Grün-Farbenblindheit", eine Farbenfehlsichtigkeit, die bei ca. 5% der Bevölkerung vorliegt. Solche Personen würden also an einem Computer oder einem ähnlichen Gerät ein Farbschema vermeiden, bei dem eine grüne Schrift auf rotem Hintergrund oder eine ähnliche Ausgabe resultieren würde. Dazu muss jedoch der betreffenden Person bewusst sein, dass sie unter einer solchen oder einer anderen Farbenfehlsichtigkeit leidet. Gerade in sicherheitsrelevanten Bereichen kann sonst die Aktivierung eines ungeeigneten Farbeschemas zu Problemen führen, wenn beispielsweise die Farben grün (häufig für die Visualisierung eines sicheren Betriebszustandes verwendet) und die Farbe rot (häufig für die Visualisierung eines unsicheren Betriebszustandes verwendet) nicht klar unterschieden werden können.

Es ist also eine Aufgabe der vorliegenden Erfindung, ein Verfahren und ein Gerät zur Anpassung der für die Anzeige eines Objektes auf eine Anzeigevorrichtung verwendeten Farben vorzuschlagen, wobei die Verwendung eines ungeeigneten Farbeschemas automatisch erkannt und korrigiert werden soll.

Die Aufgabe wird durch ein Verfahren gemäß dem Patentanspruch 1 und durch ein Gerät gemäß dem Patentanspruch 6 gelöst.

Es ist dabei eine zentrale Idee der vorliegenden Erfindung, dass nach Inbetriebnahme eines Computers oder eines anderen Gerätes dem Benutzer eine Startseite o.ä. angezeigt wird, bei der der Benutzer eine Auswahlmöglichkeit, beispielsweise eine "O.K."-Schaltfläche, betätigen muss, um das Gerät in Betrieb zu setzen bzw. mit seiner Arbeit fortzufahren. Diese Schaltfläche soll dabei in einem Farbkontrast dargestellt werden, der auch für die weiteren Bildschirmausgaben vorgesehen ist bzw. von zentraler Bedeutung ist. Sofern der Benutzer diese Schaltfläche nicht oder nur schlecht erkennen kann, soll er zur Betätigung einer anderen, für ihn sichtbaren bzw. besser sichtbaren Schaltfläche angeleitet werden. Dadurch kann eine Auswerte-Software erkennen, dass die für die Schaltfläche verwendete Farbkombination bzw. der verwendete Farbkontrast nicht geeignet ist, und in der Folge ein anderes Farbschema einstellen, bei dem eine Erkennbarkeit sichergestellt ist.

Zur Lösung der Aufgabe wird dabei insbesondere ein Verfahren zur Anpassung der für die Anzeige eines Objektes auf eine Anzeigevorrichtung verwendeten Farben vorgeschlagen, wobei für einen jeweiligen Benutzer ein für diesen geeignetes Farbschema aus einer Mehrzahl von Farbschemata verwendet wird, und wobei mittels der Farbschemata die für die Anzeige von Objekten zu verwendenden Farbkontraste jeweils festgelegt werden. Dazu wird mittels der Anzeigevorrichtung eine erste Abfragemeldung mit zumindest zwei alternativen Eingabemöglichkeiten ausgegeben, wobei der Benutzer mittels der Abfragemeldung zu einer Eingabe aufgefordert wird, und wobei eine erste der alternativen Eingabemöglichkeiten mittels eines ersten Farbkontrastes und eine zweite alternative Eingabemöglichkeit mittels eines davon abweichenden Farbkontrastes angezeigt wird. Dabei ist die Abfragemeldung derart gestaltet, dass der Benutzer zur Auswahl derjenigen der alternativen Eingabemöglichkeiten angeleitet wird, die für ihn erkennbar ist und/oder bevorzugt wird, so dass anhand der Eingabe des Benutzers ein geeignetes der Farbschemata eingestellt wird.

Zur Lösung der Aufgabe wird weiter ein Gerät mit einer graphischen Benutzeroberfläche vorgeschlagen, wobei das Gerät zur Ausführung des vorstehend beschriebenen Verfahrens ausgebildet ist.

Vorteilhafte Ausgestaltungen des erfindungsgemäßen Verfahrens sind in den abhängigen Patentansprüchen angegeben. Die dabei beschriebenen Merkmale und Vorteile gelten sinngemäß auch für das erfindungsgemäße Gerät.

Eine automatische Erkennung einer Farbsehschwäche eines Benutzers wird begünstigt, wenn die erste alternative Eingabemöglichkeit mittels eines Standard-Farbkontrastes an einer zentralen Stelle der Anzeigevorrichtung dargestellt wird, und die zweite alternative Eingabemöglichkeit mittels eines Hell-Dunkel-Kontrastes an einer nebengeordneten Stelle der Anzeigevorrichtung dargestellt wird. Dadurch wird sichergestellt, dass ein Benutzer, der keine Farbsehschwäche (Farbenfehlsichtigkeit) gegenüber der Farbkombination der ersten alternativen Eingabemöglichkeit (Schaltfläche) aufweist, diese auch betätigen wird, und nur in den Fällen, in denen der Benutzer Schwierigkeiten mit der Erkennung der ersten alternativen Eingabemöglichkeit hat, auf die zweite alternative Eingabemöglichkeit ausgewichen wird. Dadurch kann eine Farbenfehlsichtigkeit erkannt werden, so dass automatisch ein abweichendes geeigneteres Farbschema aktiviert werden kann.

Insbesondere bei Anzeigevorrichtungen in sicherheitsrelevanten Bereichen ist es von Vorteil, wenn die Abfragemeldung zumindest bei einer Anmeldung eines Benutzers an einem mittels einer graphischen Benutzeroberfläche gesteuerten Gerät ausgegeben wird. Somit wird vermieden, dass ein solches Gerät mit einem ungeeigneten Farbschema betrieben wird.

Eine wiederholte Überprüfung des Farbschemas bezogen auf ein- und denselben Benutzer wird vermieden, wenn das mittels der Abfragemeldung als geeignet ermittelte Farbschema gespeichert und für zukünftige Benutzungen des Benutzers verwendet wird.

Ausführungsbeispiele des erfindungsgemäßen Verfahrens werden nachfolgend anhand der Zeichnungen erläutert. Sie dienen gleichzeitig der Erläuterung eines erfindungsgemäßen Gerätes.

Dabei zeigen:
- Figur 1: ein Ausführungsbeispiel eines Anmeldebildschirms mit alternativen Eingabemöglichkeiten, und
- Figur 2: eine Ausgabemeldung eines Gerätes, mit der auf die Auswahl einer zweiten alternativen Eingabemöglichkeit reagiert wird.

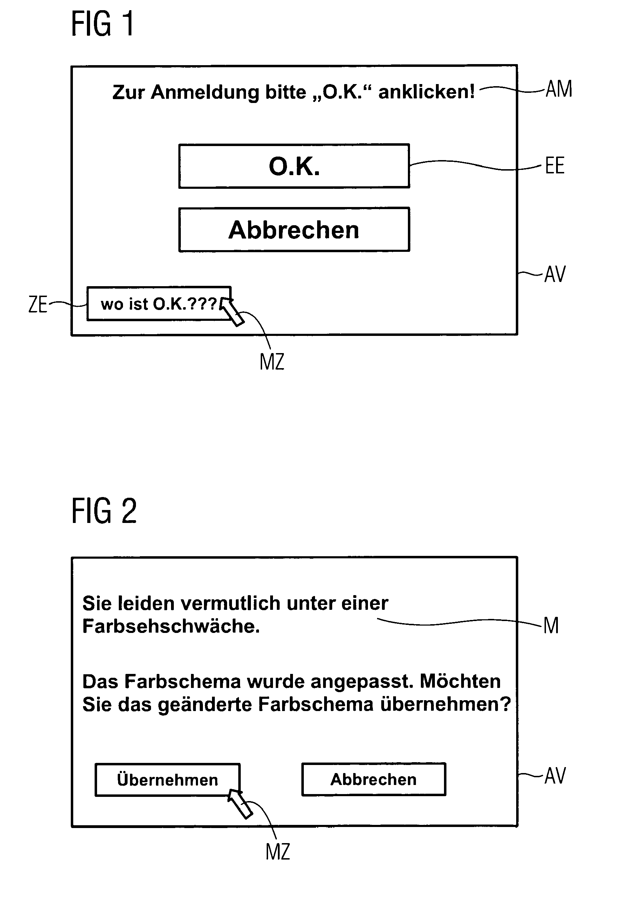

Im Folgenden wird die Einstellung (Änderung) eines geeigneten Farbschemas für einen Benutzer mit einer Farbsehschwäche am Beispiel eines Personal-Computers als Gerät beschrieben. Dazu wird nach dem "Hochfahren" des Computers auf dem Monitor AV ("Anzeigevorrichtung") eine sogenannte "Anmeldeseite" dargestellt, welche in der Figur 1 schematisch abgebildet ist. Dabei werden im vorliegenden Ausführungsbeispiel drei Eingabemöglichkeiten ("Buttons") dargeboten. Eine Textzeile AM fordert dabei den Benutzer auf, die Schaltfläche EE mit dem Mouse-Zeiger MZ zu betätigen. Dazu ist die Schaltfläche EE in einem für die weitere Bedienung des Computers vorgesehenen wichtigen Farbkontrast gestaltet, hier: Hintergrundfarbe grün, Schriftfarbe für den "O.K."-Schriftzug: rot. Dabei ist die Helligkeit der roten und der grünen Bestandteile der Schaltfläche EE vorzugsweise einander angeglichen, so dass die Lesbarkeit des Schriftzuges "O.K." allein oder zumindest vorwiegend durch den Farbkontrast gegeben ist.

Im vorliegenden Ausführungsbeispiel kann der Schriftzug "O.K." der Schaltfläche EE durch den Benutzer aufgrund seiner Farbsehschwäche nicht gelesen werden. In einem bevorzugten Ausführungsbeispiel, in dem der gesamte Bildschirm-Hintergrund des auf den Monitor AV ausgegebenen Bildes mit der Hintergrundfarbe der Schaltfläche EE übereinstimmt (hier: grün), wird der Benutzer die Schaltfläche EE überhaupt nicht erkennen können. Neben der zweiten, obligatorischen "Abbrechen"-Schaltfläche, die vorzugsweisein einem herkömmlichen Hell-Dunkel-Kontrast gestaltet ist (beispielsweise grauer Hintergrund, schwarze Schrift), wird mittels der Anzeigevorrichtung, dem Monitor AV, eine dritte Eingabemöglichkeit angezeigt, nämlich die Schaltfläche ZE, die ebenfalls wie die "Abbrechen"-Schaltfläche mit einem Hell-Dunkel-Kontrast oder in einem anderen Farbkontrast als die Schaltfläche EE gestaltet ist und durch die Beschriftung "Wo ist O.K???" dem Benutzer, der die Schaltfläche EE nicht erkennen kann, als Ziel zum "Anklicken" angeboten wird. Nun betätigt der Benutzer diese Schaltfläche ZE, wodurch mittels der Software-Steuerung des Gerätes detektiert wird, dass der für die Schaltfläche EE derzeit verwendete Farbkontrast vermutlich ungeeignet ist. Das Gerät wählt also nun ein abweichendes Farbschema, in welchem der bislang für die Schaltfläche EE verwendete Farbkontrast (hier: rot/grün) nicht für wichtige Angaben vorgesehen ist, sondern durch einen anderen Farbkontrast oder durch einen Hell-Dunkel-Kontrast (oder Beides) ersetzt wird. Im ersteren Fall der Ersetzung durch einen anderen Farbkontrast kann nun optional die in der Figur 1 dargestellte Anmeldeseite erneut dargestellt werden, wobei jedoch diesmal für die Schaltfläche EE ein anderer Farbkontrast gewählt wird, beispielsweise blau/gelb. Durch diesen optionalen Zwischenschritt kann sichergestellt werden, dass das geänderte Farbschema für wichtige Bildschirmausgaben geeignet ist.

Sobald durch die Durchführung der bislang geschilderten Verfahrensschritte ein geeignetes Farbschema aufgefunden wurde, wird die in der Figur 2 dargestellte Meldung M ausgegeben, wodurch der Benutzer die automatisiert vorgenommene Auswahl bestätigen kann, wobei durch das Drücken der "Abbrechen"-Schaltfläche ein (hier nicht dargestellter) Dialog zur manuellen Änderung der für die Anzeigevorrichtung zur verwendenden Farben bzw. das zu verwendende Farbschema ausgegeben wird.

Optional kann vor Ausgabe des in der Figur 1 gezeigten Anmelde-Bildschirms auch eine manuelle Abfrage geschaltet werden, bei der der Benutzer nach einer ihm bekannten Farbsehschwäche befragt wird bzw. dem Benutzer bereits einige vordefinierte Farbschemata zur manuellen Auswahl dargeboten werden. In diesem Fall dient das oben anhand der Figur 1 geschilderte Verfahren zur Verifizierung der Auswahl, d.h. zu einer Kontrolle, ob das gewählte Farbschema für den Benutzer und unter den derzeitigen Einsatzbedingungen (Bildschirmeinstellungen, Umgebungslicht etc.) geeignet ist. Selbstverständlich können auch mit ein- und demselben Anmeldebildschirm durch Verwendung mehrerer Schaltflächen nach Art der Schaltfläche EE mehrere Farbkombinationen zugleich hinsichtlich ihrer Eignung überprüft werden.

## Patentansprüche

1. Verfahren zur Anpassung der für die Anzeige eines Objektes auf einer Anzeigevorrichtung verwendeten Farben, wobei für einen jeweiligen Benutzer ein für diesen geeignetes Farbschema aus einer Mehrzahl von Farbschemata verwendet wird, wobei mittels der Farbschemata die für die Anzeige von Objekten zu verwendenden Farbkontraste jeweils festgelegt werden,
**dadurch gekennzeichnet,**
**dass** mittels der Anzeigevorrichtung eine erste Abfragemeldung mit zumindest zwei alternativen Eingabemöglichkeiten ausgegeben wird, wobei der Benutzer mittels der Abfragemeldung zu einer Eingabe aufgefordert wird, wobei eine erste der alternativen Eingabemöglichkeiten mittels eines ersten Farbkontrastes und eine zweite alternative Eingabemöglichkeit mittels eines davon abweichenden Farbkontrastes angezeigt wird,
wobei die Abfragemeldung derart gestaltet ist, dass der Benutzer zur Auswahl derjenigen der alternativen Eingabemöglichkeiten angeleitet wird, die für ihn erkennbar ist und/oder bevorzugt wird, und
**dass** anhand der Eingabe des Benutzers ein geeignetes der Farbschemata eingestellt wird.

2. Verfahren nach Patentanspruch 1,
**dadurch gekennzeichnet,**
**dass** die erste alternative Eingabemöglichkeit mittels eines Standard-Farbkontrastes an einer zentralen Stelle der Anzeigevorrichtung dargestellt wird, und
**dass** die zweite alternative Eingabemöglichkeit mittels eines Hell-Dunkel-Kontrastes an einer nebengeordneten Stelle der Anzeigevorrichtung dargestellt wird.

3. Verfahren nach einem der vorhergehenden Patentansprüche,
**dadurch gekennzeichnet,**
**dass** die Abfragemeldung zumindest bei einer Anmeldung eines Benutzers an einem mittels einer graphischen Benutzeroberfläche gesteuerten Gerätes ausgegeben wird.

4. Verfahren nach Patentanspruch 3,
**dadurch gekennzeichnet,**
**dass** das mittels der Abfragemeldung als geeignet ermittelte Farbschema gespeichert und für zukünftige Benutzungen des Benutzers verwendet wird.

5. Verfahren nach einem der vorhergehenden Patentansprüche,
**dadurch gekennzeichnet,**
**dass** das Verfahren zur Detektion einer Farbsehschwäche des Benutzers und für die Aktivierung eines diesbezüglich geeigneten Farbschemas verwendet wird.

6. Gerät mit einer graphischen Benutzeroberfläche,
mit einer zur Wiedergabe farblicher Inhalten geeigneten Anzeigevorrichtung und mit einer Eingabevorrichtung,
**dadurch gekennzeichnet,**
**dass** es zur Ausführung eines der vorstehend beschriebenen Verfahren ausgebildet ist.
